# EUROPÄISCHE PATENTSCHRIFT

(11) **EP 2 502 899 B1**
(45) Veröffentlichungstag und Bekanntmachung des Hinweises auf die Patenterteilung: **12.08.2015**
(21) Anmeldenummer: 12159467.5
(22) Anmeldetag: 14.03.2012
(51) Int. Cl.: C07C 67/333, C07C 67/313, C07C 69/48, C07C 69/716, C07C 51/285, C07C 53/126, C07C 55/02, C07C 51/373, C07C 51/34, C07C 59/147

(54) **Synthese von alpha, omega-Dicarbonsäuren und deren Estern aus ungesättigten Fettsäurederivaten**
Synthesis of alpha, omega-dicarboxylic acids and their esters from unsaturated fatty acid derivatives
Synthèse d'acides alpha et oméga-dicarboniques et de leurs esters à partir de dérivés d'acides gras insaturés

(30) Priorität: 25.03.2011 DE 102011015150
(43) Veröffentlichungstag der Anmeldung: 26.09.2012
(73) Patentinhaber: Evonik Degussa GmbH, 45128 Essen (DE)
(72) Erfinder: Hannen, Peter, 45701 Herten (DE); Häger, Harald, 59348 Lüdinghausen (DE); Roos, Martin, 45721 Haltern am See (DE)

(56) Entgegenhaltungen:
- DE-A1- 3 715 464
- GB-A- 841 653

## Beschreibung

Die Erfindung betrifft ein Verfahren zur Herstellung von alpha, omega-Dicarbonsäuren oder deren Estern durch Ozonolyse und anschließende Oxidation.

Unter Ozonolyse im Sinne der Erfindung wird die Spaltung von einer Kohlenstoff-Kohlenstoff-Doppelbindung durch Einwirkung von Ozon verstanden. Je nach Aufarbeitungsweise erhält man Carbonylverbindungen, Alkohole oder Carbonsäuren.

Unter alpha, omega Dicarbonsäuren werden Carbonsäuren mit zwei Carboxylgruppen verstanden, wobei die Kohlenstoffkette an Position 1 und an der endständigen Position mit einer Carboxylgruppe substituiert ist.

Die Ozonolyse von Olefinen stellt eine wichtige Methode zur Herstellung von Carbonsäuren, Aldehyden und Alkoholen dar (Baily, P.S., Ozonation in Organic Chemistry, Academic: New York, NY, 1978, Vol. 1.). Kern dieses Reaktionstyps ist die 1,3-dipolare Cycloaddition von Ozon an eine C,C-Doppelbindung eines Olefins (1) unter Bildung des Primärozonids (1,2,3-Trioxolan, 2). Bei dieser Spezies handelt es sich um ein instabiles Intermediat, das unmittelbar in ein Aldehydfragment (3) und ein Carbonyloxid (4) zerfällt (Schema 1).

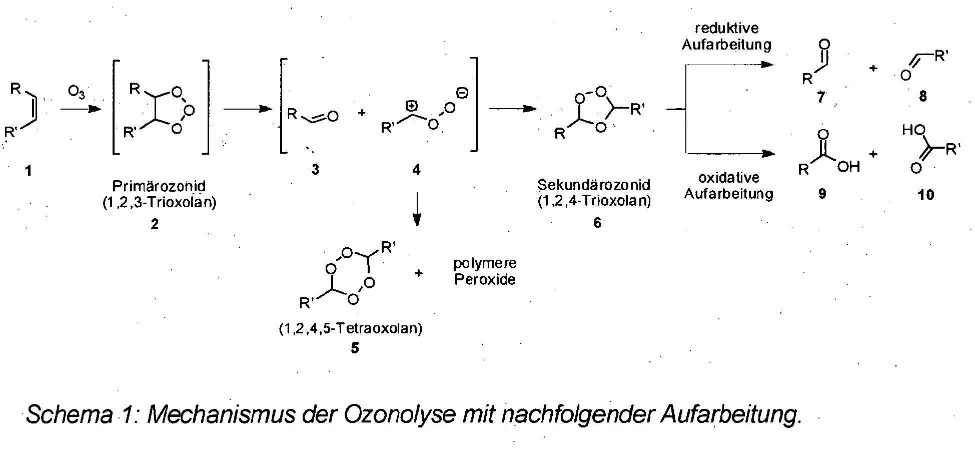

Das Carbonyloxid kann zum einen polymerisieren bzw. zu einem 1,2,4,5-Tetraoxolan (5) dimerisieren, oder in einer weiteren Cycloaddition zu einem Sekundärozonid (1,2,4-Trioxolan, 6) rekombinieren. Ausgehend von Verbindung 6 lassen sich Aldehyde (7, 8) über eine reduktive bzw. Carbonsäuren (9, 10) über eine oxidative Aufarbeitung herstellen (Kropf, H., Houben-Weyl Methoden Der Organischen Chemie; Kropf H. Ed.; Georg Thieme: Stuttgart, 1988; Vol. E13/2, S.1111.; Smith, M.B., March, J.March's Advanced Organic Chemistry; John Wiley & Sons, Inc; 2001, 5. Ed., S. 1522). Die Aldehyde wiederum können weiter bis zum Alkohol reduziert werden.

Wesentlicher Nachteil dieser Reaktionssequenz ist die Bildung der meist explosiven Sekundärozonide, polymeren Peroxide bzw. 1,2,4,5-Tetraoxolane, die z. T. stabile Verbindungen darstellen und sich so in nachfolgenden Reaktions- und Aufarbeitungsschritten anreichern können und eine erhebliche Gefährdung darstellen (Kula, J. Chem. Health Saf. 1999, 6, 21; Gordon, P.M. Chem. Eng. News 1990, 68, 2). Des Weiteren müssen bei einer oxidativen bzw. reduktiven Aufarbeitung von Sekundärozoniden ein Oxidations- bzw. Reduktionsäquivalent eingesetzt werden (Sauerstoff, Wasserstoffperoxid bzw. Dimethylsulfid, Triphenylphosphin etc.).

Ein weiteres Problem tritt bei der Oxidationsreaktion auf. Wird die Oxidation bei niedrigeren Temperaturen durchgeführt, verläuft sie sehr langsam, eine Erhöhung der Temperatur führt jedoch zu einer verstärkten Bildung von Nebenprodukten, die in weiteren Verfahrensschritten aufwendig entfernt werden müssen. Hierzu gehört z. B. Kettenabbau durch Decarboxylierung.

Aus GB 841653 A ist bekannt, dass ungesättigte Fettsäuren durch Ozonolyse und anschließender Oxidation zu mono- und dibasischen Säuren umgesetzt werden können. Als Oxidationsmittel wird Sauerstoff eingesetzt.

DE 3715464 A1 beschreibt die Gewinnung von Diperazelainsäure aus einem Gemisch ungesättigter Fettsäuren durch Fettspaltung, Destillation, oxidativer Ozonolyse des so erhaltenen Fettsäuregemisches und Umsetzung der so erhaltenen Azelainsäure mit Wasserstoffperoxid zu Diperazelainsäure.

Die technische Aufgabe der vorliegenden Erfindung ist es daher, ein verbessertes Verfahren zur Herstellung von alpha,omega-Dicarbonsäuren oder deren Estern zur Verfügung zu stellen, das die Bildung von Ozoniden vermeidet und eine direkte Umsetzung des Reaktionsproduktes aus der Ozonolyse für die Oxidation ermöglicht.

Eine weitere technische Aufgabe ist es das Verfahren so zu modifizieren, dass die Oxidation der aus der Ozonolyse erhaltenen Reaktionsgemische beschleunigt wird.

Diese technischen Aufgaben werden gelöst durch ein Verfahren zur Herstellung von alpha,omega-Dicarbonsäuren oder deren Estern gekennzeichnet durch die folgenden Schritte
a. Ozonolyse ungesättigter Fettsäuren oder Fettsäurederivate,
b. Oxidation des aus der Ozonolyse erhaltenen Reaktionsgemisches zur alpha, omega Dicarbonsäuren oder deren Estern, wobei im Schritt b) als Katalysator eine starke Säure mit einem pKs-Wert von kleiner gleich null gemessen bei 25°C zugegeben wird, und
   wobei das Verfahren unter Verwendung eines Lösungsmittels durchgeführt wird.

Es wurde überraschend gefunden, dass alpha,omega-Dicarbonsäuren bzw. deren Ester leicht in einem Schritt über eine Ozonolyse und anschließende Oxidation gebildet werden können. Dabei ist wesentlich, dass die Bildung der Dicarbonsäuren über intermediär gebildete Aldehyde und nicht über die Oxidation von Sekundärozoniden erfolgt.

Bei dem erfindungsgemäßen Verfahren fallen im Ozonolyseschritt keine polymeren und oligomeren Ozonide an. Ein wesentlicher Aspekt der beschriebenen Erfindung ist die deutliche Beschleunigung der Oxidation durch den Zusatz katalytischer Mengen einer starken Säure.

Das erfindungsgemäße Verfahren ist als eine Kombination aus Ozonolyse und Oxidation anzusehen. Dabei wird das Reaktionsgemisch der Ozonolyse ohne Aufarbeitung den Bedingungen einer weiteren Oxidation zum Dicarbonsäurederivat unterzogen.

In einer bevorzugten Ausführungsform wird als Katalysator eine Säure eingesetzt ausgewählt aus der Gruppe bestehend aus Schwefelsäure, Salzsäure, Salpetersäure, Perchlorsäure oder Mischungen davon. Der Einsatz von konzentrierter Schwefelsäure oder Perchlorsäure ist besonders bevorzugt.

Bei der Reaktion können grundsätzlich verschiedene Lösungsmittel verwendet werden. So ist es bekannt, dass durch Verwendung einer Lösung von 5 bis 10 Gew.-% Wasser in Aceton über eine Ozonolyse aus Ölsäuremethylester selektiv Nonanal und 9-Oxonanansäuremethylester hergestellt werden kann (Dussault, P.H.,Journal of Chemistry 2008, 73, 4688-4690).

In dem erfindungsgemäßen Herstellungsverfahren können neben Aceton auch andere Lösungsmittel wie beispielsweise Methylethylketon, Alkohole (wie Isopropanol, tert.-Butanol etc.) oder Carbonsäuren mit vergleichbar guten Ergebnissen eingesetzt werden.

In bevorzugter Ausführungsform wird als Lösungsmittel eine aliphatische Carbonsäure im Gemisch mit mindestens 0,5 Gew.-% Wasser, bezogen auf die Gesamtmenge an Lösungsmittel, eingesetzt.

In besonders bevorzugter Ausführungsform enthält das Lösungsmittel 1 bis 20 Gew.-% Wasser, ganz besonders bevorzugt 2 bis 15 Gew.-% Wasser, bezogen auf die Gesamtmenge an Lösungsmittel.

In besonders bevorzugter Ausführungsform ist der Anteil an Wasser im Reaktionsgemisch mindestens in stöchiometrischer Menge vorhanden zur Anzahl der umgesetzten Doppelbindungen der Fettsäure oder des Fettsäurederivats.

In bevorzugter Weise wird als Lösungsmittel eine C₁-C₁₅-, besonders bevorzugt C₃-C₁₀-aliphatische Carbonsäure eingesetzt. Ganz besonders bevorzugt sind Propionsäure und/oder Essigsäure.

Als Fettsäure oder deren Ester mit mindestens einer Doppelbindung werden bevorzugt ungesättigte Fettsäuren oder Fettsäurederivate, ausgewählt aus der Gruppe Ölsäure, Ölsäurealkylester, Undecylensäure, Undecylensäurealkylester, Erucasäure, Erucasäurealkylester oder Mischungen davon eingesetzt.

In bevorzugter Ausführungsform wird der Oxidationsschritt b. bei einer Temperatur von kleiner gleich 110 °C, besonders bevorzugt von kleiner gleich 100 °C durchgeführt. Das Verfahren kann kontinuierlich oder diskontinuierlich durchgeführt werden.

Als Oxidationsmittel wird bei der Reaktion vorzugsweise Wasserstoffperoxid und/oder eine Peroxocarbonsäure eingesetzt. Ein Äquivalent Wasserstoffperoxid pro gespaltener Doppelbindung entsteht bei der Ozonolyse durch Reaktion des Wassers mit dem Primärozonid und kann dann besonders bevorzugt im Oxidationsschritt eingesetzt werden. Peroxocarbonsäuren sind ebenfalls bevorzugt, weil sich diese aus dem Wasserstoffperoxid zusammen mit den als Lösungsmittel vorhandenen Carbonsäuren bilden kann.

Das Verfahren zeichnet sich durch seine, im Vergleich mit bekannten Verfahren, sichere Durchführung aus. Ozonide bzw. das intermediär entstehende Carbonyloxid werden direkt von Wasser abgefangen. Das als Hydroperoxid 11 bezeichnete Addukt aus Ozonid und Wasser liegt im Gleichgewicht mit Aldehyd 12 und Wasserstoffperoxid vor (Schema 2).

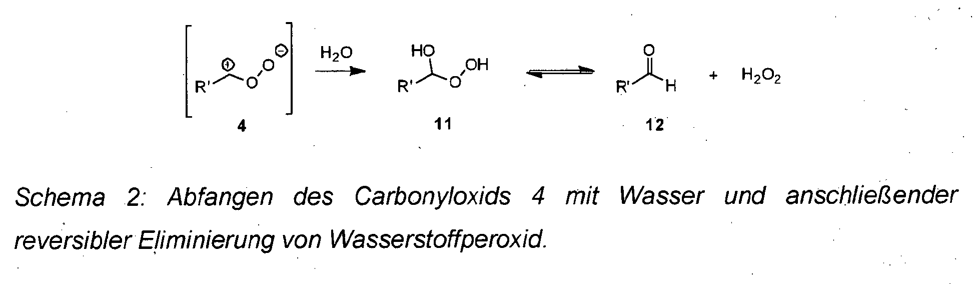

Die Bildung von Wasserstoffperoxid bei der Ozonolyse kann nun besonders vorteilhaft bei einer oxidativen Aufarbeitung genutzt werden. Neben der schon erwähnten Vermeidung der Bildung gefährlicher Ozonide, wird bei der Reaktion direkt ein Oxidationsäquivalent gebildet.

Das in Schema 2 gezeigte Gleichgewicht kann unter Einstellung passender Reaktionsbedingungen genutzt werden, um eine Carbonylgruppe zur Carboxylgruppe zu oxidieren. Wie in Schema 3 gezeigt, addiert Wasserstoffperoxid zunächst reversibel unter Bildung von Hydroperoxid 13 an Aldehyd 3. Unter Abspaltung eines Hydrogeniumions wird aus Addukt 13 irreversibel die Carboxylverbindung 9 gebildet. Diese Reaktion verläuft bevorzugt bei höheren Temperaturen von 100 bis 200 °C, vorzugsweise von 150 °C.

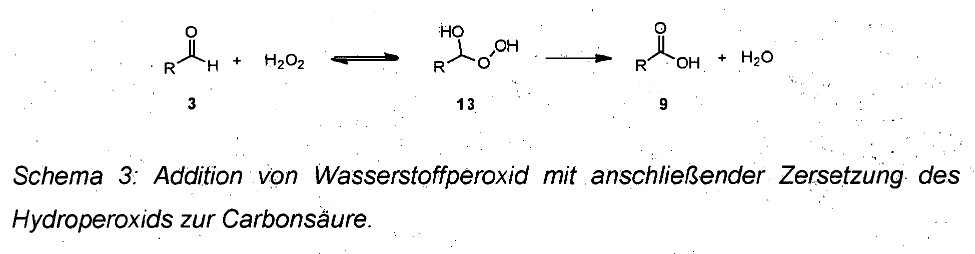

Die Oxidation bei niedrigeren Temperaturen unter 100 °C verläuft ohne Katalysatoren vergleichsweise langsam und eine Erhöhung der Temperatur kommt aufgrund der verstärkten Bildung von Nebenprodukten nicht in Frage.

Es wurde gefunden, dass die Zugabe katalytischer Mengen einer Säure die Zersetzung des Hydroperoxids und damit die Oxidation insgesamt stark beschleunigt. Zum einen konnte dadurch die Reaktionsdauer stark verkürzt werden, zum anderen ist damit einhergehend nun eine Reaktion bei deutlich niedrigeren Temperaturen möglich. Die Bildung unerwünschter Nebenprodukte durch eine lange Reaktionszeit bei hoher Temperatur kann dadurch vermieden werden. Es wird angenommen, dass durch die Protonierung der Hydroperoxidgruppe die Zersetzung begünstigt wird (Schema 4).

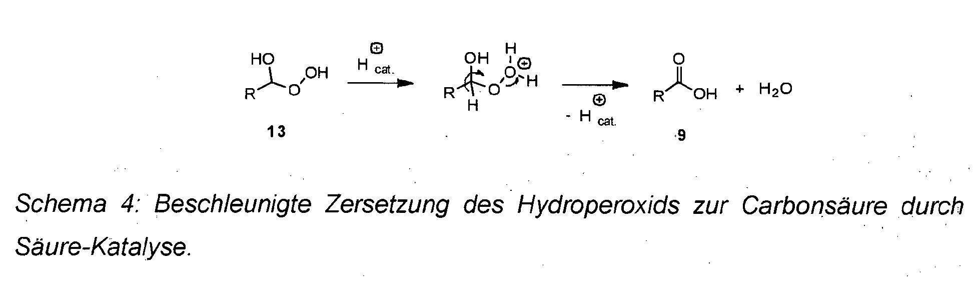

Einen deutlichen katalytischen Effekt zeigen bei der beschriebenen Reaktion allerdings nur Säuren mit einem ausreichend kleinen pKₛ Wert von kleiner gleich 0, gemessen bei 25 °C.

So wird die Reaktion beispielsweise durch katalytische Mengen an konz. Schwefelsäure oder Perchlorsäure stark beschleunigt. Besonders bevorzugt ist allerdings der Einsatz von Schwefelsäure als Katalysator.

Neben der in Schema 4 gezeigten direkten Reaktion der Carbonylgruppe mit Wasserstoffperoxid zur Carboxylgruppe ist unter den Reaktionsbedingungen auch eine Oxidation durch eine Peroxocarbonsäure denkbar. Die Peroxocarbonsäure bildet sich unter Säure-Katalyse aus der als Lösungsmittel eingesetzten Carbonsäure und Wasserstoffperoxid. Die Peroxocarbonsäure addiert an die Carbonylgruppe und zerfällt wieder unter Bildung zweier Carboxylgruppen.

Um beide Fragmente der Ozonolyse (Schema 1, Verbindungen 3 und 4) zur entsprechenden Carbonsäure zu oxidieren, wird nun ein weiteres Oxidationsäquivalent benötigt. In dem erfindungsgemäßen Verfahren wird dies besonders einfach durch die Zugabe eines weiteren Äquivalents Wasserstoffperoxid erreicht, wodurch eine vollständige Oxidation zu den Carbonsäuren erfolgt (Schema 5).

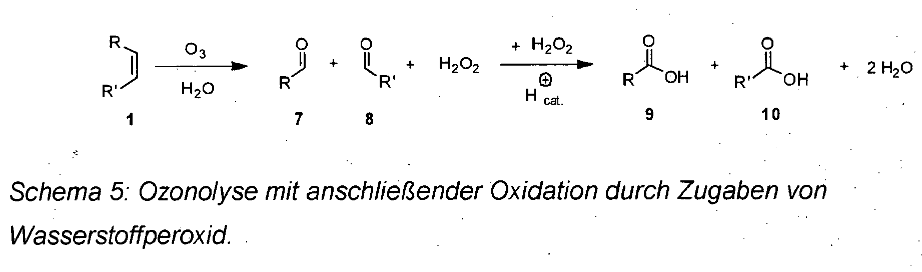

Der Einsatz von Wasserstoffperoxid als Oxidationsmittel hat zudem den Vorteil, dass es sich gut dosieren lässt und nach der Reaktion lediglich Wasser als Zersetzungsprodukt entsteht. Dies sind für ein technisches Verfahren entscheidende Vorteile gegenüber z. B. der Verwendung von Luftsauerstoff als Oxidationsmittel, da das Reaktionssystem einphasig vorliegt, sich keine explosionsfähigen Gasgemische bilden und keine aufwändige Abtrennung von mitgerissenem Reaktionsgemisch im Abgasstrom stattfinden muss. Die Oxidation kann batchweise oder kontinuierlich durchgeführt werden. Bei einer kontinuierlichen Oxidation wird bevorzugt ein Kapillarreaktor verwendet, bei dem ein guter Wärmeaustausch gegeben ist.

Ein weiterer Vorteil der säurekatalysierten Oxidation liegt in der gleichzeitigen Spaltung der Estergruppe bei Einsatz entsprechender Fettsäureester. Dadurch liegt eines der Produkte nicht als Dicarbonsäuremonoester sondern als Dicarbonsäure vor, was die Aufarbeitung des Reaktionsgemisches deutlich vereinfacht.

Als Fettsäuren oder Fettsäurederivate werden solche mit mindestens einer Doppelbindung eingesetzt. Dabei sind als Fettsäuren und Fettsäurederivate insbesondere bevorzugt Verbindungen ausgewählt aus der Gruppe Ölsäure, Ölsäurealkylester, Undecylensäure, Undecylensäurealkylester, Erucasäure, Erucasäurealkylester oder Mischungen davon.

Als Ausgangsprodukte für das erfindungsgemäße Verfahren können aber auch andere ungesättigte Fettsäuren oder Fettsäurederivate eingesetzt werden. Hierzu gehören beispielsweise Myristoleinsäure, Palmitoleinsäure, Petroselinsäure, Elaidinsäure, Vaccensäure, Gadoleinsäure, Icosensäure, Cetoleinsäure und Nervonsäure und deren Ester. Hierbei handelt es sich um einfach ungesättigte Fettsäuren. Weiterhin können auch mehrfach ungesättigte Fettsäuren eingesetzt werden wie beispielsweise Linolsäure, Linolensäure, Calendulasäure, Punicinsäure, Elaeostearinsäure, Arachidonsäure, Timnodonsäure, Clupanodonsäure und Cervonsäure oder deren Ester.

Das erfindungsgemäße Verfahren wird wie folgt durchgeführt. Die Ozonolyse wird zunächst in einem Lösungsmittel durchgeführt, das bevorzugt eine Carbonsäure ist. Besonders geeignet sind beispielsweise Propionsäure, Essigsäure oder Pelargonsäure. Der ungesättigte Fettsäureester liegt in einer Konzentration von 0,1 bis 1 mol/L vor. Bei den höheren Konzentrationen an Fettsäureestern muss beachtet werden, dass die Menge an zugesetztem Wasser immer mindestens stöchiometrisch zur Anzahl der umgesetzten Doppelbindungen vorliegt. Das Reaktionsgemisch liegt bevorzugt einphasig vor. Prinzipiell kann die Reaktion aber auch mit einem mehrphasigen Reaktionsgemisch erfolgen. Die Ozonolyse wird bevorzugt im Temperaturbereich von 0 bis 50 °C durchgeführt, besonders bevorzugt ist eine Reaktionstemperatur von 20 bis 30 °C.

Als Ozonolyse im Sinne der Erfindung wird die Reaktion einer Fettsäure oder eines Fettsäurederivates mit Ozon verstanden.

Üblicherweise nutzt man zur Ozonerzeugung einen Ozongenerator. Dieser Ozongenerator verwendet als Speisegas technische Luft oder ein Gemisch aus Kohlendioxid und Sauerstoff. In dem Ozongenerator wird das Ozon durch stille elektrische Entladung hergestellt. Dabei werden Sauerstoffradikale gebildet, die sich mit Sauerstoffmolekülen zu Ozon umsetzen.

Das aus der Ozonolyse erhaltene Reaktionsgemisch kann ohne weitere Aufarbeitung und Abtrennung dem Oxidationsschritt unterzogen werden. Hierzu wird ein Oxidationsmittel verwendet, das bevorzugt Wasserstoffperoxid oder eine Peroxycarbonsäure ist. Bezogen auf die Anzahl der Doppelbindungen im Substrat (Fettsäure oder Fettsäurederivat) wird ein weiteres Äquivalent an Oxidationsmittel zugesetzt und das Gemisch auf Temperaturen im Bereich von kleiner gleich 110 °C, vorzugsweise kleiner gleich 100 °C erhitzt. Ganz besonders bevorzugt wird die Reaktion in einem Temperaturbereich von 90 bis 110 °C durchgeführt. Die Oxidation kann auch bei tieferen Temperaturen durchgeführt werden, wobei hier jedoch die Reaktion langsamer verläuft. Bei Temperaturen oberhalb von 110 °C ist die Bildung der Reaktionsprodukte zwar beschleunigt, aber es entsteht eine höhere Konzentration an Nebenprodukten.

Die Reaktionssequenz Ozonolyse und Oxidation lässt sich kontinuierlich durchführen, wobei die Ozonolyse bevorzugt in einem Rieselbettreaktor stattfindet, in dem die Reaktanden im Gegenstrom über eine Füllkörperschüttung geführt werden. Dem Ozonolyseteil direkt angeschlossen ist ein Kapillarreaktor, in dem die Oxidation erfolgt. Zwischen Ozonolyse- und Oxidationsteil der Apparatur wird dem Reaktionsgemisch kontinuierlich das Oxidationsmittel, bevorzugt Wasserstoffperoxid, zudosiert.

Das erfindungsgemäße Verfahren weist die folgenden Vorteile auf:
Sekundäre und oligomere Ozonide werden durch den Zusatz von Wasser vermieden. Hierdurch wird das erfindungsgemäße Verfahren sicherer gestaltet, da diese Substanzen explosiv sind.

Ein weiterer Vorteil liegt darin, dass bei dem erfindungsgemäßen Verfahren direkt die Bildung von je einem Äquivalent der zwei möglichen Aldehyd-Zwischenprodukte erfolgt und einem Äquivalent Wasserstoffperoxid, das bei der weiteren Reaktion für die Oxidation eingesetzt werden kann.

Ein weiterer Vorteil ist es, dass die Oxidation vollständig erfolgt, wobei die als Zwischenprodukt entstehenden Aldehyde durch Zugabe nur eines zusätzlichen Äquivalents Wasserstoffperoxid zu den entsprechenden Carbonsäuren reagieren, während das andere Äquivalent durch die Reaktion bei der Ozonolyse entsteht.

Weiterhin wird die Reaktion beschleunigt durch katalytische Zugabe einer starken Säure und kann somit wirtschaftlicher durchgeführt werden.

Die nachfolgenden Beispiele sollen die Erfindung näher erläutern.

### Beispiele

### Beispiel 1 - Vergleichsbeispiel

### Ozonolyse und Oxidation ohne Säurezugabe

20 g einer 0,182 molalen Lösung von Ölsäuremethylester (95 % rein) in einem Lösungsmittelgemisch aus Propionsäure und Wasser (15 Äquivalente bezogen auf Mole Doppelbindung) werden in einem Zweihalskolben mit Gaseinleitungsrohr und Rückflusskühler vorgelegt. Das Speisegas, bestehend aus 5 Vol-% Sauerstoff in Kohlendioxid wird mit einer Durchflussgeschwindigkeit von 40 mL/min. durch einen Ozongenerator geleitet. Der Ozongenerator ist dabei auf maximale Leistung gestellt. Das Ozon-haltige Gasgemisch wird unter Rühren in das Reaktionsgemisch geleitet. Der Abgasstrom wird über Gaswaschflaschen in eine 5 %-ige, wässrige Kaliumiodid-Lösung geleitet. Nach 60 Minuten ist das Substrat umgesetzt, worauf die Gaseinleitung unterbrochen wird. Das Reaktionsgemisch hat laut GC-Analyse einen Gehalt von 39,5 Gew.-% 9-Nonanal und 38,2 Gew.-% 9-Oxo-nonansäuremethylester.

Das Reaktionsgemisch wird dann nach Zugabe von Wasserstoffperoxid (0,454 g einer 30 %-igen, wässrigen Lösung) in einem Ölbad auf 100 °C erwärmt. Nach 120 Minuten sind Nonanal bzw. 9-Oxononansäuremethylester vollständig zu den jeweiligen Carboxylverbindungen umgesetzt. GC-Analyse: 41,05 % Pelargonsäure, 39,65 % Azelainsäuremonomethylester (FID Signal, Angabe in Flächenprozent unkorrigiert)

### Beispiel 2

### Ozonolyse und Oxidation mit Säurezugabe

20 g einer 0,182 molalen Lösung von Ölsäuremethylester (95 % rein) in einem Lösungsmittelgemisch aus Propionsäure und Wasser (15 Equivalente bezogen auf Mole Doppelbindung) werden in einem Zweihalskolben mit Gaseinleitungsrohr Rückflusskühler vorgelegt. Das Speisegas, bestehend aus 5 Vol-% Sauerstoff in Kohlendioxid wird mit einer Durchflussgeschwindigkeit von 40 mL/min. durch einen Ozongenerator geleitet. Der Ozongenerator ist dabei auf maximale Leistung gestellt. Das Ozon-haltige Gasgemisch wird unter Rühren in das Reaktionsgemisch geleitet. Der Abgasstrom wird über Gaswaschflaschen in eine ca. 5 %-ige, wässrige Kaliumiodid-Lösung geleitet. Nach 60 Minuten ist das Substrat umgesetzt, worauf die Gaseinleitung unterbrochen wird. Das Reaktionsgemisch hat laut GC-Analyse einen Gehalt von 39,5 % 9-Nonanal und 38,2 % 9-Oxo-nonansäuremethylester.

Das Reaktionsgemisch wird nach Zugabe von Wasserstoffperoxid (0,454 g einer 30 %-igen, wässrigen Lösung) und Schwefelsäure (0,019 g, 95 %-ig) in einem Ölbad auf 100 °C erwärmt. Nach 75 Minuten sind Nonanal bzw. 9-Oxo-nonansäuremethylester vollständig zu den jeweiligen Carboxylverbindungen umgesetzt. GC-Analyse: 40,22 % Pelargonsäure, 38,50 % Azelainsäurederivat (21,90 % Azelainsäure-mono-methylester + 16,6 % Azelainsäure) (FID Signal, Angabe in Flächenprozent unkorrigiert)

## Patentansprüche

1. Verfahren zur Herstellung von alpha, omega Dicarbonsäuren oder deren Estern **gekennzeichnet durch** die folgenden Schritte:
a. Ozonolyse ungesättigter Fettsäuren oder Fettsäurederivate,
b. Oxidation des aus der Ozonolyse erhaltenen Reaktionsgemisches zu alpha, omega Dicarbonsäuren oder deren Estern, wobei im Schritt b) als Katalysator eine starke Säure mit einem pKs-Wert von kleiner gleich null gemessen bei 25°C zugegeben wird, und
wobei das Verfahren unter Verwendung eines Lösungsmittels durchgeführt wird.

2. Verfahren nach Anspruch 1, **dadurch gekennzeichnet, dass** der Katalysator ausgewählt wird aus der Gruppe bestehend aus Schwefelsäure, Salzsäure Salpetersäure, Perchlorsäure oder Mischungen davon.

3. Verfahren nach Anspruch 1 oder 2 **dadurch gekennzeichnet, dass** das Verfahren mit einer aliphatischen Carbonsäure im Gemisch mit mindestens 0,5 Gew.- % Wasser als Lösungsmittel bezogen auf die Gesamtmenge an Lösungsmittel durchgeführt wird.

4. Verfahren nach Anspruch 1 bis 3, **dadurch gekennzeichnet, dass** das Lösungsmittel 1 bis 20 Gew.-% Wasser bezogen auf die Gesamtmenge an Lösungsmittel enthält.

5. Verfahren nach Anspruch 1 bis 4, **dadurch gekennzeichnet, dass** das Lösungsmittel 2 bis 15 Gew.-% Wasser bezogen auf die Gesamtmenge an Lösungsmittel enthält.

6. Verfahren nach den Ansprüchen 1 bis 5, **dadurch gekennzeichnet, dass** das Lösungsmittel 5 bis 10 Gew.-% Wasser bezogen auf die Gesamtmenge an Lösungsmittel enthält, aber mindestens in stöchiometrischer Menge vorliegt zur Anzahl der umgesetzten Doppelbindungen der Fettsäure oder des Fettsäurederivats.

7. Verfahren nach den Ansprüchen 1 bis 6, **dadurch gekennzeichnet, dass** Fettsäuren oder Fettsäurederivate mit mindestens einer Doppelbindung eingesetzt werden.

8. Verfahren nach den Ansprüchen 1 bis 7, **dadurch gekennzeichnet, dass** die Ozonolyse (Schritt a)) und die Oxidation (Schritt b)) direkt nacheinander ohne Isolierung oder Aufarbeitung des Reaktionsgemisches aus der Ozonolyse durchgeführt werden.

9. Verfahren nach den Ansprüchen 1 bis 8, **dadurch gekennzeichnet, dass** als ungesättigte Fettsäuren oder Fettsäurederivate Verbindungen ausgewählt aus der Gruppe Ölsäure, Ölsäurealkylester, Undecylensäure, Undecylensäurealkylester, Erucasäure, Erucasäurealkylester eingesetzt werden.

10. Verfahren nach den Ansprüchen 1 bis 9, **dadurch gekennzeichnet, dass** als Lösungsmittel eine C₁ bis C₁₅ aliphatische Carbonsäure, vorzugsweise eine C₃ bis C₁₀ aliphatische Carbonsäure verwendet wird.

11. Verfahren nach den Ansprüchen 1 bis 10, **dadurch gekennzeichnet, dass** als Lösungsmittel Propionsäure und/oder Essigsäure verwendet wird.

12. Verfahren nach den Ansprüchen 1 bis 11, **dadurch gekennzeichnet, dass** die Oxidation (Schritt b)) bei einer Temperatur von kleiner gleich 110°C, vorzugsweise kleiner gleich 100°C durchgeführt wird.

13. Verfahren nach den Ansprüchen 1 bis 12, **dadurch gekennzeichnet, dass** das Verfahren kontinuierlich oder batchweise durchgeführt wird.

14. Verfahren nach den Ansprüchen 1 bis 13, **dadurch gekennzeichnet, dass** im Oxidationsschritt b) Wasserstoffperoxid und/oder eine Peroxocarbonsäure als Oxidationsmittel eingesetzt wird.

## Claims

1. Process for preparing alpha,omega-dicarboxylic acids or esters thereof, **characterized by** the following steps:
a. ozonolysing unsaturated fatty acids or fatty acid derivatives,
b. oxidizing the reaction mixture obtained from the ozonolysis to give alpha, omega-dicarboxylic acids or esters thereof, with addition in step b) of a strong acid catalyst with a pKa of less than or equal to zero, measured at 25°C, and
wherein the process is performed using a solvent.

2. Process according to Claim 1, **characterized in that** the catalyst is selected from the group consisting of sulphuric acid, hydrochloric acid, nitric acid, perchloric acid and mixtures thereof.

3. Process according to Claim 1 or 2, **characterized in that** it is performed with an aliphatic carboxylic acid in a mixture with at least 0.5% by weight of water as a solvent, based on the total amount of solvent.

4. Process according to Claims 1 to 3, **characterized in that** the solvent contains 1 to 20% by weight of water, based on the total amount of solvent.

5. Process according to Claims 1 to 4, **characterized in that** the solvent contains 2 to 15% by weight of water, based on the total amount of solvent.

6. Process according to Claims 1 to 5, **characterized in that** the solvent contains 5 to 10% by weight of water, based on the total amount of solvent, but is present at least in a stoichiometric amount relative to the number of converted double bonds of the fatty acid or fatty acid derivative.

7. Process according to Claims 1 to 6, **characterized in that** fatty acids or fatty acid derivatives with at least one double bond are used.

8. Process according to Claims 1 to 7, **characterized in that** the ozonolysis (step a)) and the oxidation (step b)) are performed in direct succession without isolating or working up the reaction mixture from the ozonolysis.

9. Process according to Claims 1 to 8, **characterized in that** the unsaturated fatty acids or fatty acid derivatives used are compounds selected from the group of oleic acid, oleic acid alkyl esters, undecylenoic acid, undecylenoic acid alkyl esters, erucic acid, erucic acid alkyl esters.

10. Process according to Claims 1 to 9, **characterized in that** the solvent used is a C₁ to C₁₅ aliphatic carboxylic acid, preferably a C₃ to C₁₀ aliphatic carboxylic acid.

11. Process according to Claims 1 to 10, **characterized in that** the solvent used is propionic acid and/or acetic acid.

12. Process according to Claims 1 to 11, **characterized in that** the oxidation (step b)) is performed at a temperature of less than or equal to 110°C, preferably less than or equal to 100°C.

13. Process according to Claims 1 to 12, **characterized in that** it is performed continuously or batchwise.

14. Process according to Claims 1 to 13, **characterized in that** hydrogen peroxide and/or a peroxocarboxylic acid is used as an oxidizing agent in the oxidation step b).

## Revendications

1. Procédé pour la préparation d'acides alpha, oméga dicarboxyliques ou leurs esters, **caractérisé par** les étapes suivantes :
a. ozonolyse d'acides gras insaturés ou de dérivés d'acides gras insaturés,
b. oxydation du mélange réactionnel obtenu à partir de l'ozonolyse en acides alpha, oméga dicarboxyliques ou leurs esters, un acide fort présentant une valeur pKa inférieure ou égale à zéro, mesurée à 25°C, étant ajouté dans l'étape b) comme catalyseur, et
le procédé étant réalisé en utilisant un solvant.

2. Procédé selon la revendication 1, **caractérisé en ce que** le catalyseur est choisi dans le groupe constitué par l'acide sulfurique, l'acide chlorhydrique, l'acide nitrique, l'acide perchlorique ou leurs mélanges.

3. Procédé selon la revendication 1 ou 2, **caractérisé en ce que** le procédé est réalisé avec un acide carboxylique aliphatique en mélange avec au moins 0,5% en poids d'eau comme solvant, par rapport à la quantité totale de solvant.

4. Procédé selon la revendication 1 à 3, **caractérisé en ce que** le solvant contient 1 à 20% en poids d'eau, par rapport à la quantité totale de solvant.

5. Procédé selon la revendication 1 à 4, **caractérisé en ce que** le solvant contient 2 à 15% en poids d'eau, par rapport à la quantité totale de solvant.

6. Procédé selon les revendications 1 à 5, **caractérisé en ce que** le solvant contient 5 à 10% en poids d'eau, par rapport à la quantité totale de solvant, mais se trouve au moins en quantité stoechiométrique par rapport au nombre de doubles liaisons transformées de l'acide gras ou du dérivé d'acide gras.

7. Procédé selon les revendications 1 à 6, **caractérisé en ce qu'**on utilise des acides gras ou des dérivés d'acides gras présentant au moins une double liaison.

8. Procédé selon les revendications 1 à 7, **caractérisé en ce que** l'ozonolyse (étape a)) et l'oxydation (étape b)) sont réalisées directement l'une après l'autre sans isolement ni traitement du mélange réactionnel provenant de l'ozonolyse.

9. Procédé selon les revendications 1 à 8, **caractérisé en ce qu'**on utilise, comme acides gras insaturés ou dérivés d'acides gras insaturés, des composés choisis dans le groupe formé par l'acide oléique, les esters alkyliques de l'acide oléique, l'acide undécylénoïque, les esters alkyliques de l'acide undécylénoïque, l'acide érucasique, les esters alkyliques de l'acide érucasique.

10. Procédé selon les revendications 1 à 9, **caractérisé en ce qu'**on utilise, comme solvant, un acide carboxylique aliphatique en C₁ à C₁₅, de préférence un acide carboxylique aliphatique en C₃ à C₁₀.

11. Procédé selon les revendications 1 à 10, **caractérisé en ce qu'**on utilise, comme solvant, de l'acide propionique et/ou de l'acide acétique.

12. Procédé selon les revendications 1 à 11, **caractérisé en ce que** l'oxydation (étape b)) est réalisée à une température inférieure à 110°C, de préférence inférieure à 100 °C.

13. Procédé selon les revendications 1 à 12, **caractérisé en ce que** le procédé est réalisé en continu ou par lots.

14. Procédé selon les revendications 1 à 13, **caractérisé en ce qu'**on utilise, dans l'étape d'oxydation b), du peroxyde d'hydrogène et/ou un acide peroxocarboxylique comme oxydant.
